# EUROPEAN PATENT APPLICATION

(11) **EP 2 179 739 A1**
(43) Date of publication of application: **28.04.2010**
(21) Application number: 08018597.8
(22) Date of filing: 23.10.2008
(51) Int. Cl.: A61K 36/87, A61K 31/341, A61P 17/00

(54) **Compositions comprising vitamins**

(71) Applicant: Tutino, Matteo, Palermo (IT)
(72) Inventor: Tutino, Matteo, Palermo (IT)
(74) Representative: Rothkopf, Ferdinand

(57) **Abstract**

Compositions according to the invention comprise one or more vitamins and/or hormones and/or alpha arbutine and/or their derivatives in association with an Vitis Vinifera extract, preferably an aqueous extract, and/or one or more of its components, such as resveratrol, polyphenols and anthocyanidine, and/orionene polimers, along with one or more adjuvants and/or excipients pharmaceutically or cosmetically acceptable.

## Description

The present invention relates to compositions comprising vitamins. Particularly, the invention concerns stable compositions with high vitamin concentration, such as ascorbic acid, preferably L-ascorbic acid, and/or their derivatives, to be advantageously employed in the medical and cosmetic fields, for example for the cosmetic and dermatological treatment of skin and mucous membranes.

L-ascorbic acid, i. e. vitamin C, is widely employed in the medical field and particularly in the alimentary field, this substance being fundamental for the human organism. In fact, lack of this vitamin induces in human being a disease known as "scorbutus". Being vitamin C fundamental for collagen, in the last decades it has raised a remarkable interest in the pharmaceutical cosmetic sector. Furthermore, it is known the application of vitamin C as antimicrobial and anti-oxidant additive for nutritional food, or as purifying and disinfectant for pharmaceutical preparations such as cosmetics.

However, ascorbic acid in a serum solution has the tendency to the quick natural decomposition, even when it remains perfectly dissolved. L-ascorbic acid is readily soluble in water, but it quickly oxidises in aqueous solutions, and thus it cannot be stabilised with a sufficient concentration in this natural solvent. On the other hand, solubility of L-ascorbic acid in aqueous solutions is very limited. Instability of L-ascorbic acid in aqueous solutions is due to its alpha-ketonic structure, to its interaction with water, to unavoidable penetration of oxygen in the solution where vitamin has been dissolved, to the effect of the exposition to the light and to the time.

During the years different ways for preparing vitamin C (L-ascorbic acid) solutions stable for a reasonably long time have been suggested.

Particularly, International Patent Application WO98/23152 shows various preparation and action methods of vitamin C, both in its dextrogyrate form (ascorbyl palmitat) and in its levogyrate form (LAA), the latter being considered the form needed by the cell for activating its metabolism.

Particularly, stabilisation of vitamin C is described solubilizing vitamin in a solvent, such as polyethylene glycol, ethoxydiglycol, propylene glycol, butylene glycol, propylene carbonate, glycerine, capric or caprylic glyceride, alkyl lactate, alkyl adipate, isosorbide, and their mixtures.

However, known methods do not allow obtaining L-ascorbic acid compositions with a sufficient intracellular penetration.

In view of the above, it is an object of the invention to provide new alternative methods and compositions able to stabilise vitamins, particularly vitamin C, solving the drawbacks of the known compositions.

The object is solved by compositions comprising one or more vitamins and/or hormons and/or alpha arbutine and/or their derivatives in association with a Vitis Vinifera extract, preferably an aqueous extract, and/or one or more of its components, such as resveratrol, polyphenols and anthocyanidine, and/or ionene polymers, along with one or more adjuvants and/or excipients pharmaceutically or cosmetically acceptable.

In a first preferred embodiment said vitamins and/or their derivatives are ascorbic acid and/or its derivatives.

In a second preferred embodiment said ascorbic acid is L-ascorbic acid, and/or derivatives of ascorbic acid are esters of ascorbic acid with fatty acids or their salts, and/or ester of ascorbic acid with fatty acids is ascorbyl palmitate.

In a third preferred embodiment vitamins are chosen in the group comprising vitamin A, E, D, K, B1, B2, B3, B5, B6, B8, B9, B12, and/or wherein hormons are chosen in the group comprising melatonin, αMSH, testosteron, estrogens, pregnenolon, calcitonin, somatostatin.

In a forth preferred embodiment alpha arbutine is stabilized with melatonin, polyphenols, resveratrol, Vitis Vinifera, ascorbic acid and phytosphingosine, and/or the compositions further comprising Echinacea purpurea extract, angustifolia or pallida and/or one or more active principles contained in Echinacea purpurea extract, angustifolia or pallida, such as heteroxylanes, rhamnoarabino-galactanes, glycoproteines, cichoric acid, alkylamides, caffeic acid derivatives, flavonoids, caryophyllene and green tea.

In a fifth preferred embodiment derivatives of caffeic acid are echinacoside or Echinacea and/or flavonoid is rutin.

In a sixth preferred embodiment the compositions further comprising one or more compounds chosen from the group comprising polymeric ethers, monohydric alcohols, polyhydric alcohols, tetraborates or thiosulfates.

In a seventh preferred embodiment polymeric ethers are ethylene polymers or propylene oxides and/or wherein polyhydric alcohol is ethoxydiglycol.

In an eight preferred embodiment the composition further comprising one or more compounds chosen in the group comprising adenosine triphosphate, adenosine diphosphate, phosphoenolpyruvate, creatine phosphate, inorganic phosphate, and/or further comprising one or more substances included in the group comprising collagen, in particular type I collagen, fibrin glue, fibrin and its derivatives, dura mater, and/or further comprising one or more compounds chosen from the group comprising hyaluronic acid or its derivatives, hydroxymethylcellulose, maltodextrines, amino acids.

In a ninth preferred embodiment the compositions being in the form of serum, gel emulsions, creams, medical devices, such as gauzes, bandages, plasters, silicon bars.

In a tenth preferred embodiment ionene polymers have the general formula: [-N(CH₃)₂-(CH₂)ₓ-N(CH₃)₂-(CH₂)_{y}]*2Z - wherein x and y are integral numbers and Z is an halide, such as Br or Cl and/or wherein ionene polymer is obtained by reaction of 1,4-dichlorobutane with tetra-methylen-diamine.

In a eleventh preferred embodiment ionene polymers are chosen among those obtained by reaction of 1, 4-dichlorobutane with poly (oxyethylene(dimethylimino)-ethylene (dimethylimino) ethylene dihalides), poly (2-hydroxyethylene-dimethyliminio-2-hydroxypropylene- dimethyliminomethylene) dihalides, poly [{alkyl-(3- ammoniopropyl)imino} trimethylene dihalides], poly-[dialkyl-imino) ethylene halides] or with poly-[(hydroxy-dialkyl-imino) ethylene halides.

In a twelfth preferred embodiment ascorbic acid or its derivatives are present at a concentration between 0.1 % and 35 % or preferably between 10 % and 25 % or more preferably between 12 % and 20 %.

In a thirdteenth preferred embodiment Vitamin A is present at a concentration between 0.01 % and 5 %.

In a fourteenth preferred embodiment Vitamin E is present at a concentration between 0.01 % and 5 %.

In a fifteenth preferred emobodiment ionene polymers are present at a concentration between 0.001 % and 10 % or between 1.5 % and 3.2 %.

In a sixteenth preferred emobiment extract of Vitis Vinifera is present at a concentration between 0.1 % and 95 %, or preferably between 5 % and 50 % or more preferably between 15 % and 30 %, and/or polymeric ethers are present at a concentration between 5 % and 50 %, and/or mono- and/or polyhydric alcohols are present at a concentration between 5 % and 50 %, and/or thiosulfates are present at a concentration between 0 % and 5 %, preferably between 0.01 % and 3 %, and/or Echinacea purpurea and/or one or more of its active principles are present at a concentration between 0.001 % and 10 %, and/or one or more compounds of the group comprising adenosine triphosphate, adenosine diphosphate, phosphoenolpyruvate, creatine phosphate, inorganic phosphate are present at a concentration between 0.001 % and 35 %.

In a seventeenth preferred embodiment thiosulfates are provided as potassium and/or sodium and/or ammonium salts.

In a eighteenth preferred embodiment the composition further comprising 20 % water, preferably bi-distilled water, 20 % L-ascorbic acid, 2.66 % resveratrol and melatonin, 0.3 % potassium thiosulfate, 15 % aqueous extract of Vitis Vinifera, 42 % of a mixture 50 : 50 of propylenglycol and 1,2-butylenglycol, or comprising 20 % water, preferably bi-distilled water, 20 % L-ascorbic acid, 2.66 % ionene polymer having formula(I), with x=3 and y=3 , 0.3% potassium thiosulfate, 22% aqueous extract of Vitis Vinifera, 15 % polyoxyethylene/polyoxypropylene polymer, 20 % of a mixture 50 : 50 of propylenglycol and 1,2-butylenglycol, or comprising 15 % L-ascorbic acid, 0,34 % resveratrol, 26.5 % aqueous extract of Vitis Vinifera, 44 % ethoxydiglycol, 0.06 % citric acid, 15 % depurated water, or comprising 15 % L-ascorbic acid, resveratrol and melatonin, 15 % aqueous extract of Vitis Vinifera, 44 % ethoxydiglycol, 0.06 % citric acid, 15 % depurated water.

In a nineteenth preferred embodiment the compositions having a pH value in the range between 2.0 and 6.0.

In a twentieth preferred embodiement the compostions further vitamin A at a concentration between 0.01 % and 5 % and vitamin E at a concentration between 0.01 % and 5 % in association with one or more ionene polymers, present at a concentration between 0.001 % and 10 %, preferably between 1.5 % and 3.2 %, along with one or more adjuvants and/or excipients, pharmaceutically or cosmetically acceptable.

In a twentiethfirst preferred embodiment the compositions further comprising one or more compounds chosen in the group comprising cogic acid, azelaic acid, fitic acid, glycolic acid, lactic acid, fumaric acid, tartaric acid, L-aspartic acid, L-ascorbic acid, Arbutin, and/or said one or more components are present at a concentration between 1.5 % and 10 %.

In a twentiethsecond preferred embodiment the compostions further comprising one or more emollients, flowing, emulsifying agents, preservatives, surface-active agent, fungicides, and in particualr wherein emollient are cetyl esters at a concentration between 0.1 % and 10 %, preferably between 5 % and 9 %, and/or wherein flowing are chosen between stearyl alcohol at a concentration between 0. 1 % and 15 %, preferably between 5 % and 12 %, cheryl alcohol at a concentration between 5 % and 12 %, and/or wherein emulsifying agents are chosen among cetyl alcohol at a concentration between 0.1 % and 6 % or between 2 % and 5 %, glycerine between 0.1 % and18 % or between 2 % and 12 %, laurylsulfate sodium between 0.1 % and 1.5 % or 0,5 % and 1.0 %, and/or wherein preservative is methylparaben at a concentration between 0.1 % and 0.4 % or 0.05 % and 0.3 %, and/or wherein surface-active agent is present at a concentration between 0.01 % and 0.15 % or 0.05 % and 0,12 %, and/or wherein fungicide is propyl paraben at a concentration between 0.01 % and 0.1 % or 0.02 % and 0.05 %, and/or Phenoxyethanol, Methylchloroisothiazolinone, Metylisothiazolinone at a concentration from 0.01 % to 0.50 % and/or phytosphingosine from 0.1 % to 5.0 %.

In a twentiethird preferred embodiment the compostions further comprising ionene polymer between 0.01 % and 10 % in weight, cogic acid between 1 % and 10 % in weight, azelaic acid between 1 % and 30 % in weight, glycolic and/or lactic acid between 1.5 % and 15 % in weight, trans-cis retinic acid between 0.01 % and 5 % in weight, acetate E vitamin between 0.5 % and 5 % in weight, hydrating base cream comprising at least an emollient or wetting-agent selected from a group comprising cetyl esters, cetyl alcohol, glycerine, α-preservative selected from the group comprising methyl paraben, propyl paraben and laurylsulfate sodium as emulsifying agent, and finally water up to 100% in weight, and/or Phenoxyethanol, Methylchloroisothiazolinone, Metylisothiazolinone, as preservatives.

In a twentiethforth preferred embodiment the compositions being for use in the medical field or for cosmetic treatment, such as treatment of wrinkles and/or treatment of skin spots, and/or preventive antiageing treatment.

In a twentiethfifth preferred embodiment the compostions being for use for preparation of a medicament for treatment of keratosis actinic and/or for treatment of wounds and/or for treatment of sores and/or for treatment of diabetic cutaneous sores, and of acute and chronic lesions of skin and/or for treatment of lesions of oral mucous and/or for prevention and treatment of skin tumours and/or for treatment of psoriasis and/or for treatment of hair growth as skin tone balancing cream and/or as modulator of the TNF and inflammatory processes and/or for treatment of the post inflammatory hyperpigmentation melasma and/or for treatment of laser complications.

The object is futher solved by use of resveratrol and/or melatonin and/or ionene polymers and/or Vitis Vinifera for stabilising compositions comprising vitamins, and/or wherein compositions are aqueous solutions or emulsions and/or wherein vitamins are chosen from the group comprising vitamin A, B1, B2, B3, B5, B6, B8, B9, B12, C, D, E, K and/or aminoacids as well as oligopeptides.

The applicant of the present invention has now found that some compounds usually employed separately in the pharmaceutical, dermatological and cosmetic formulations, such as Vitis Vinifera, resveratrol, melatonin, polyphenols and anthocianidines, sometimes in presence of polymeric ethers, tetraborates or thiosulfate, allow stabilising L-ascorbic acid in aqueous compositions. The above mentioned compounds allow preparing stable compositions with higher concentration of L-ascorbic acid in such a way of making said compositions more efficient, improving the intracellular, intranuclear, intra-mitochondrial penetration and optimising the bio-availability approaching the intracellular saturation. Furthermore, association between L-ascorbic acid and Vitis Vinifera and/or resveratrol and/or melatonin and/or polyphenols extract develops a synergistic effect between the compounds of the composition thus able to increase the efficiency of each component.

Aqueous extract of Vitis Vinifera has been rarely used up to date as cosmetic topic treatment. This substance is a strong antioxidant and a microbicide, and, furthermore, by some peculiar substances, among which polyphenols, anthocianidines etc., exerts an important anti-tumorous action. Efficiency of substances contained in the aqueous extract of Vitis Vinifera, (resveratrol and polyphenols) are employed in natural medicine and for treatment of some dermatological and cardiovascular affections. Said substance is some times used for exerting a regulation action of the immunitary system, also in case of allergy. At present, vitamin C, resveratrol, polyphenols and melatonin have never been used in combination with Vitis Vinifera with ascorbic acid.

Vitis Vinifera, melatonin and L-ascorbic acid (LAA) have important biological functions for skin and all the cellular tissues:
- they have an antioxidant action opposing to "oxygen free" radicals stimulated by the same cellular metabolism and by the smoke, by the ultraviolet light exposition and by other polluting attacks, and they oppose to the cellular ageing;
- have an anti-inflammatory action due to a strengthening of the immunitary system;
- reinforce the cellular response to the outer and intracellular nociceptive stimuli;
- exert a noticeable action in the modulation of the immunitary response;
- intervene with an important antioxidant action in the body zones where an alteration of the cellular growth having a tumorous origin, a dysplastic alteration and/or a tissue atrophy is present;
- stimulate synthesis of collagen in fibroblasts of human skin
- and particularly L-ascorbic acid (LAA) intervenes in homeostasis of connective system of human organism.
- Furthermore, L-ascorbic acid (LAA) acts for increasing synthesis of proteins and collagen, with anti-wrinkles effects, chelating action with respect to ferric ions, prevention of skin damages due to an excessive exposition to the sun (J Invest Dermatol. 1997, Radial Res. 2003).

L-ascorbic acid, Vitis Vinifera, resveratrol, melatonin and polyphenols play an important role in treatment and prevention of cancer, and this is due to the particular action of some substances contained in the Vitis Vinifera, such us resveratrol, polyphenols, etc... combined with vitamin C, causing vitamin having a direct intracellular, intra-mitochondrion and citoplastic action.

By the term "Echinacea" different species of endemic plants from North America are indicated. Echinacea is from the Compositae family, and in the present classification of the Echinacea kind nine species and two varieties are indicated. However, only E. purpurea, E. angustifolia and E. pallida are used in therapy.

Results of many pharmacological studies have demonstrated that the various preparations that can be obtained by the aerial parts and by the roots of the medicinal plants of the Echinacea kind have the capability of stimulating the activity of the immunitary system, strengthening the functions of the natural killer cells and cyto-toxicity antibodies-dependent of the mononuclear cells of peripheral blood. Chemical components responsible of said pharmaceutical activity are a plurality: mainly polysaccharides (heteroxylanes and rhamno-arabino-galactanes), glycoproteins, cichoric acid, alkylamides, caffeic acid derivatives (echinacoside, Echinacea), terpenes, flavonoids (from leaves), rutin, caryophyllene. Further pharmaceutical effects of Echinacea are antiviral, bacteriostatic, fungistatic, anti-inflammatory, cicatrising activities.

It is therefore a specific object of the present invention to provide compositions comprising one or more vitamins and/or their derivatives in association with an Vitis Vinifera extract, preferably an aqueous extract, and/or one or more of its components, such as resveratrol, polyphenols, anthocianidines, and/or one or more ionene polymers, along with one or more adjuvants and/or excipients pharmaceutically active or cosmetically acceptable.

Derivatives of ascorbic acid according to the present invention can be esters of ascorbic acid with fatty acids as, for example, ascorbyl palmitate and their salts.

Further vitamins, either hydro- and liposoluble vitamins, that can be present in the compositions according to the present invention are chosen in the group comprising vitamin A, B1, B2, B3, B5, B6, B8, B9, B12, D, E, K.

Compositions according to the invention can further comprise Echinacea purpurea extract, angustifolia or pallida and/or one or more active principles contained therein, such as, for example, heteroxylanes, rhamno-arabino-galactanes, glycoproteines, cichoric acid, alkylamides, caffeic acid derivatives such as, for example, echinacoside and Echinacea, terpenes, flavonoids such as for example rutin, caryophyllene. In fact, extract of Echinacea or its active principles, besides having pharmaceutical activity, can further stabilise vitamins, particularly vitamin C.

Further, compositions can comprise one or more compounds chosen from the group comprising polymeric ethers, for example ethylene polymers or propylene oxides, monohydric alcohols, poly-hydric alcohols, for example etoxydiglycole, for example Transcutol®" also known as APV, tetraborates or thiosulfates.

According to a particular embodiment of the present invention, compositions can comprise one or more compounds chosen in the group comprising adenosine triphosphate, adenosine diphosphate, phosphoenolpyruvate, creatine phosphate, and inorganic phosphate. These compounds represent a source of cellular energy for generation of a proton gradient.

Above mentioned compositions can comprise also one or more substances included in the group comprising collagen, particularly type I collagen, fibrin glue, fibrin and its derivatives, dura mater. Association of ascorbic acid or its derivatives with one or more of the above substances is particularly advantageous in the treatment of wounds. In fact, these components are topic chemotactic agents of neutrophil,fibroblasts and/or endothelial cells. Above mentioned combination can be used in medical devices, such as gauzes, bandages, plasters, silicon bars, treated with the composition according to the invention or it can be employed for example as emulsion, gel, dust, paste, dispersion, solution. Wounds that can be treated with the compositions according to the invention are skin wounds, burns, sores, surgical ferrite, and chronic and acute lesions of skin and of mucous. Healing of wounds is accelerated and formation of anaesthetic cicatrix, such as keloids, hypertrophic cicatrix, is reduced, being promoted the production of type I collagen, and the cellular and extra cellular matrix regeneration processes are regulated along with all the relevant components.

Compositions according to the invention can further comprise one or more compounds chosen from the group comprising hyaluronic acid and/or its derivatives, for example esters, cross-linked or amidic derivatives, for example as gel, hydroxymethylcellulose, maltodextrines, for example in injectable form, to be used as filler. Furthermore, compositions according to the invention can comprise one or more amino acids such as glycine, alanine, valine, leucine, isoleucine, serine, cysteine threonine, methionine, phenylalanine, tyroxine, tryptofano, histidine, lysine, arginine, aspartic acid, glutamic acid, asparagine, glutamin, proline. Compositions according to the invention have a pH included in the range between 2.0 and 6.0 and, preferably, are in the serum, gel, emulsion and cream form.

As already mentioned in the above, said compositions can be used also for the preparation of gauzes, bandages, plasters, silicon bars, for example embedded with the compositions according to the invention. Therefore, a particular embodiment of the present invention is represented by medical devices comprising the compositions according to the invention. Special solution for hair grow can be prepared containing resveratrol, vitamin C, polyphenols and melatonin at a concentration from 0.01 % up to 10 %

Composition according to the invention can comprise ascorbic acid or its derivatives at a concentration between 0.1 % and 35 %, preferably between 10 % and 25 %, still more preferably between 12 % and 20 %.

Vitamin A (trans and cis-retinoic acid) can be present at a concentration between 0.001 % and 10 %, preferably between 1.5 % and 3.2 %. Extract of Vitis Vinifera and/or one or more of its components, such as resveratrol, antocianidine and flavanoli and flavani, acid cinnamici benzoici can be present at a concentration between 0.1 % and 95 %, preferably between 5 % and 50 %, still more preferably between 15 % and 30 %. Polymeric ethers can be present at a concentration between 5 % and 50 %, mono and/or poly-hydric alcohols at a concentration between 5 % and 50 %, thiosulfates at a concentration between 0 % and 5 %, preferably between 0.01 % and 3 %.

Echinacea extract and/or one or more of its active principles can be present at a concentration between 0.001 % and 10 %, as well as one or more compounds of the group comprising adenosine triphosphate, adenosine diphosphate, phosphoenolpyruvate, creatine phosphate, inorganic phosphate can be present at a concentration between 0.001 % and 35 %.

The above mentioned percentages are weight percentages of the components with respect to the weight of the composition.

Particularly, thiosulfates are provided as potassium and/or sodium and/or ammonium salts.

Thus, they are compositions comprising high concentrations of vitamins, preferably L-ascorbic acid, stabilised along with other components improving their efficiency, intracellular, intranuclear, intramitocondrial penetration, resistance to the oxidation, hydration capability, use pleasure.

A further example is represented by: 20 % water, preferably bi- distilled water, 20 % L-ascorbic acid, 2.66 % Melatonin 0.3 % Resveratrol, 22 % aqueous extract of Vitis Vinifera, 15 % polyoxyethylene/polyoxypropylene polymer, 20 % of a mixture 50 : 50 of propylenglycol and 1,2 butylenglycol.

A further specific example of composition according to the invention is represented by: 15% % L-ascorbic acid, 26. % aqueous extract of Vitis Vinifera, 44 % ethoxyglycol, 15 % depurated water.

Compositions object of the present invention can contain disinfectant additives, bactericides and DNA correctors having an intranuclear, mitochondria and cytoplasmatic action such as resveratrol, melatonin, polyphenols and enzymes and catalysers of the intracellular biochemical energetic reactions. As to the use in the medical field of estracts from Vitis Vinifera, data relevant to pharmacological and clinical tests have been widely published in international magazines.

A particular embodiment of the present invention concerns compositions comprising vitamin A and E in association with one or more derivates from Vitis Vinifera, along with one or more adjuvants and/or excipients, pharmaceutical or cosmetically acceptable. This composition is particularly suitable for treatment of skin, mucous and serosa. It is able to promote the biosynthesis of collagen, repairing cellular damages, stabilising and strengthening the action of the substances employed in the medical, dermatological and cosmetic field, promoting a perfect penetration of the complexed substances. The composition can penetrate from the extra-cellular space inside the cell and exerting the intracytoplasmatic action also acting on the biosynthesis of proteins and, at the same time, having an action within the cellular nucleus stabilising DNA, increasing the resistance to the nociceptive stimuli and, in case DNA is already damaged, promoting a quick reparation of the same.

Vitamin A (trans and cis-retinoic acid) can be present at a concentration between 0.01 % and 5 %. Also vitamin E (alpha tocopherol), for example in its acetate form, can be present at a concentration between 0.01 % and 5 %. Resveratrol and/or Melatonin can be present at a concentration between 0.001 % and 10 %, preferably between 1.5 % and 3.2 %.

The above mentioned composition can further comprise one or more compounds chosen in the group comprising cogic acid, azelaic acid, fitic acid, glycolic acid, lactic acid, fumaric acid, tartaric acid, L-aspartic acid, phytic acid, arbutine. The above mentioned compounds can be present at a concentration between 1.5 % and 20 %.

Particularly, the composition can further comprise one or more emollients, such as cetyl esters, glycerine, flowing such as stearyl alcohol, cheryl alcohol, emulsifying agents such as cetyl alcohol, glycerine, sodium lauryl sulfate, preservatives such as methylparaben, surface-active such as Quaternium/15, fungicides such as propylparaben.

Cetyl esters having a synthetic origin and in any case not distinguishable from waxes derived from natural spermaceti as far as chemical composition and properties are concerned. These esters are comprised of a mixture of esters of fatty acids containing between14 and18 atoms of Carbon along with alcohols and they can be included in the formulations as emollient or "softening agents". Cetyl esters can be present in the formulation at a concentration between 0.1 % and 10 %, preferably between 5 % and 9 %. Stearyl alcohol can be present at a concentration between 0.1 % and 15 %, preferably between 5 % and 12 %, cheryl alcohol between 5 % and 12 %, cetyl alcohol between 0.1 % and 6 %, preferably between 2 % and 6 %, glycerine between 0.1 % and18 %, preferably between 2 % and 12 %, laurylsulfate sodium between 0.1% and 1.5 %, preferably between 0.5 % and 1.0 %.

Cetyl ester, stearyl alcohol, cheryl alcohol, and glycerine form a hydrating basic cream promoting the application on the skin with positive effects, preservation of the composition is further promoted by the presence of methyl paraben between 0.1 % and 0.4 %, preferably between 0.05 % and 0.3 %, propyl paraben between 0. 01 % and 0.1 %, preferably between 0.02 % and 0.05 %, surface-active Quaternium/15 between 0. 01% and 0.15%, preferably between 0.05 % and 0.12 %.

Deionised or distilled water is present in the formulations according to the present invention as inert carrier acting as diluent and at the same time has wetting properties.

According to a particular embodiment of the present invention, the composition comprises: Melatonin 0. 01 % and 10 % in weight, cogic acid between 1 % and 10 % in weight, azelaic acid between 1 % and 30 % in weight, glycolic and/or lactic acid between 1.5 % and 15 % in weight, trans-cis retinic acid between 0.01 % and 5 % in weight, acetate E vitamin between 0.5 % and 5 % in weight, Reservatrol between 0.01% and 10% hydrating base cream comprising at least an emollient or wetting-agent selected from a group comprising cetyl esters, cetyl alcohol, glycerine, a preservative selected from the group comprising Phenoxyethanol, Methylchloroisothiazolinone, Metylisothiazolinone, as preservatives. Lauurylsulfate sodium as emulsifying agent, and finally water up to 100 % in weight.

Preferably, the above mentioned compositions are in cream form.

It is further object of the present invention the use of the compositions according to the invention for the cosmetic treatment, for example for treatment of wrinkles, of skin spots.

Compositions according to the present invention can be further advantageously used in the medical field, both the treatment of the humans and in the veterinary field.

Particularly, it is object of the present invention the use of the compositions according to the invention for the preparation of a medicament for treatment of keratosis actinic, of wounds, of sores, of diabetic cutaneous sores, of lesions of oral mucous, of psoriasis, for prevention and treatment of skin tumours in general, and of the acute and chronic lesions of skin.

Compositions according to the present invention can be used on the human body, particularly on the skin, in association with pulsed light laser technology and particularly within wavelengths between 520 and 670 nm, and more particularly 650 nm, determining a proton gradient according to the Andrè Jagendorf law.

Furthermore, the present invention concerns the use of melatonin, resveratrol and/or Vitis Vinifera extract for stabilising compositions of vitamins or their hydro-or lipo-soluble derivatives, for example in form of aqueous solutions or emulsions, particularly compositions comprising one or more vitamins, or their derivatives, chosen from the group comprising vitamin A, B1, B2, B3, B5, B6, B8, B9, B12, C, D, E, K.

Compositions according to the present invention can be prepared according to the preparation techniques well known to those skilled in the art.

The present invention will be now described, for illustrative but not limitative purposes, according to its preferred embodiments, with particular reference to the figures of the enclosed drawings and examples, wherein:
The present invention will be now described, for illustrative but not limitative purposes, according to its preferred embodiments, with particular reference to the figures of the enclosed drawings and examples, wherein:
   Fig. 1 shows a scheme of interaction between L-ascorbic acid and/or melatonin and Vitis Vinifera and/or resveratrol and polyphenols;
   Fig. 2 and 3 show the results of case 1 (hair growth);
   Fig. 4 and 5 show the results of case 2 (dystrophic sore due to radiotherapy);
   Fig. 6 and 7 show the results of case 3 (bleaching and skin tone balancing effect);
   Fig. 8 and 9 show the results of case 4 (antiageing effect);

### EXAMPLE 1: Study of stability of a composition according to the invention with respect to a comparative formulation

The following concentrations are given in weight %:
20 % bi-distilled water
20 % L-ascorbic acid
2.66 % Melatonin
0.3 % Resveratrol
37% aqueous extract of Vitis Vinifera
20% of a 50 : 50 mixture of propylene glycol and 1,2 butylene glycol.

In this formulation, active package stabilising the L-ascorbic acid is comprised of the following compounds: aqueous extract of Vitis Vinifera, wich include polyphenols, Melatonin and Resveratrol.

This solution is addressed to the pharmaceutical, dermatological, cosmetic fields, as well as to the medical-surgical and veterinary fields, and to the treatment of wounds and sores of biological tissue, and more specifically of skin.

In the latter case, for example, equine collagen, or other kind of collagen, can be impregnated with the same solution according to the present invention, and according to method already known to those skilled in the art.

It has been prepared the following comparative solution (weight percentages) 20% bi-distilled water
20% L-ascorbic acid
50% of a solution at 50% of Ethoxidiglicol and Glicol Propilenic

### COMPARATIVE TEST

| Composition according to the invention | |
|---|---|
| After 0 months | After 25 months |
| Slightly yellow colour | unchanged colour |
| Degradation 0% | Degradation 5% - 9.8% |

| Composition according to the comparative formula | |
|---|---|
| After 0 months | After 25 months |
| White colour | dark brown colour |
| Degradation 0% | Degradation 98% |

### EXAMPLE 2: Study of stability of a composition according to the invention with respect to a comparative formulation

The following concentrations are given in weight %:
20 % L-ascorbic acid
20 % bi-distilled water
2.66 % melatonin (I)
0.3 % resveratrol
30 % aqueous extract of Vitis Vinifera
42 % of a 50 : 50 mixture of propylene glycol and 1,2 butylene glycol.

In this formulation, active package stabilising the L-ascorbic acid is comprised of the following compounds: Resveratrol and Melatonin, Vitis Vinifera.

This solution is addressed to the pharmaceutical, dermatological, cosmetic fields, as well as to the medical-surgical and veterinary fields, and to the treatment of wounds and sores of biological tissue, and more specifically of skin, and subcutaneus tissue and more specifically when vascular insufficiency is associated to skin problems.

In the latter case, for example, equine collagen, or other kind of collagen, can be impregnated with the same solution according to the present invention, and according to method already known to those skilled in the art.

It has been prepared the following comparative solution (weight percentages)
20 % bi-distilled water
20 % L-ascorbic acid
50 % of a solution at 50% of Ethoxidiglicol and Glicol Propilenic

### COMPARATIVE TEST

| Composition according to the invention | |
|---|---|
| After 0 months | After 25 months |
| Slightly yellow/ RUBY colour | Slightly yellow/RUBY colour |
| Degradation 0 % | Degradation 9.5 % |

| Composition according to the comparative formula | |
|---|---|
| After 0 months | After 25 months |
| White colour | dark brown colour |
| Degradation 0 % | Degradation 98 % |

### EXAMPLE 3: Composition of L-ascorbic acid at 15 % stabilised with Vitis Vinifera and resveratrol and polyphenols

| | |
|---|---|
| L-Ascorbic acid | 15 % |
| Resveratrol | 0.34 % |
| Aqueous extract of Vitis Vinifera | 26.5 % |
| Ethoxydiglycol | 44.0 % |
| Citric acid | 0.06% |
| Depurated water | 152% |

Slightly yellow composition remains unchanged after 25 months.

### EXAMPLE 4: Composition of L-ascorbic acid at 15 % stabilised with Vitis Vinifera extract and melatonin and polyphenols

| | |
|---|---|
| L-ascorbic acid | 15 % |
| Aqueous extract of Vitis Vinifera | 15 % |
| Melatonin | 3% |
| Resveratrol | 3% |
| Ethoxydiglycol | 44.0 % |
| | |
| Depurated water | 15 % |

Slightly ruby composition remains unchanged after 25 months.

### EXAMPLE 5: Clinical studies on results of the application of composition according to example 3 and/or 4

Different clinical applications of the compositions according to the present invention have been carried out, said compositions being based on LAA stabilised by aqueous extract of Vitis Vinifera and creams containing vitamin E, A and its derivatives, all complexed with resveratroland/or melatonin, including cogic or azelaic acid. Practically, clinical application was carried out on 15 subjects affected by various cutaneous alterations, such as: bedsores, diabetic sores, various cases of cutaneous tumours from dysplasia to actinic keratosis (pre-cancerous), thermal and chemical burns, cutaneous ageing, surgical cicatrix and other kinds of cicatrix, different kind of acne, cutaneous atrophy, cutaneous dehydration, psoriasis and other dermatological pathologies, with interesting results; therefore, some random cases are listed in the following among the 15 cases subjected to treatment.

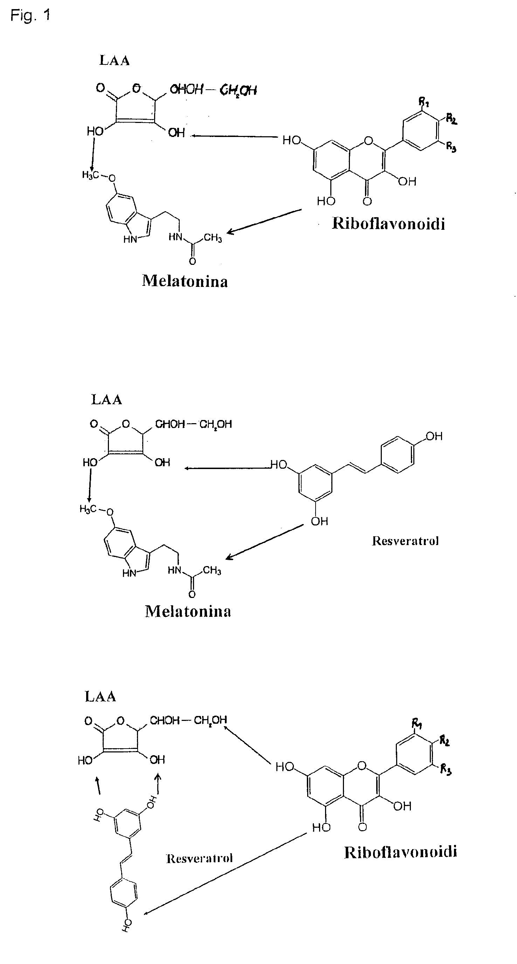

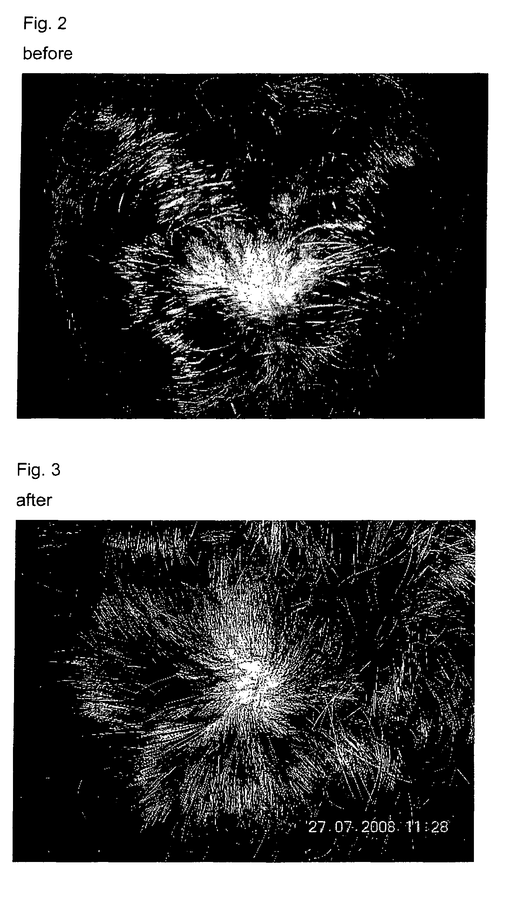

Exemplificative case n° 1: 47 year-old patient affected by alopecia areata of scalp. The patient is subjected to topical treatment with Shampoo containing melatonin 0.1 % Vitis Vinifera extracts and resveratrol togehther with vitamin C. Then, daily medications are made on the part subjected to intervention employing LAA 15% complexed with resveratrol, melatonin, polyphenols and gliceric estracts of red grapes in serum form. Medications are carried out also in the area comprising the whole scalp, since affected by cutaneous atrophy zones. After 3 months, it is noted a remarkable hair regrowth. Healing of the area is completed within 18 months from the intervention. Prosecuting the treatment with stabilised LAA and vitamin A based creams and its derivatives, complexed with Vitis Vinifera oily estracts, the perfect restoring of the whole surface affected by missing hair. Cutaneous surface treated after three months has assumed the normal aspect (Fig.1 and 2).

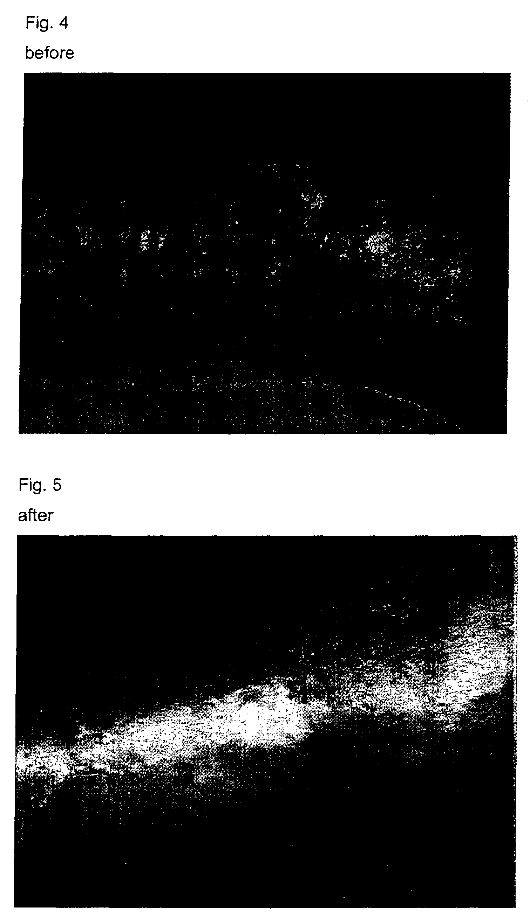

Exemplificative case n° 2: A 42 year old patient with a post irradiated wound in the leg area f; is daily medicated withLAA complexed with resveratrol and melatonin 0.5% and fatty gauzes. After seven days, the complete reepithelialization and restoring of sensitivity is obtained (Fig. 3 and 4). Patient has treated the irradiated part with the serum according to the present invention, twice a day, and after a period of 7 days, not only sores were completely reepithelialized, but also even more an important anti-inflammatory action of the part subjected to treatment was noted.

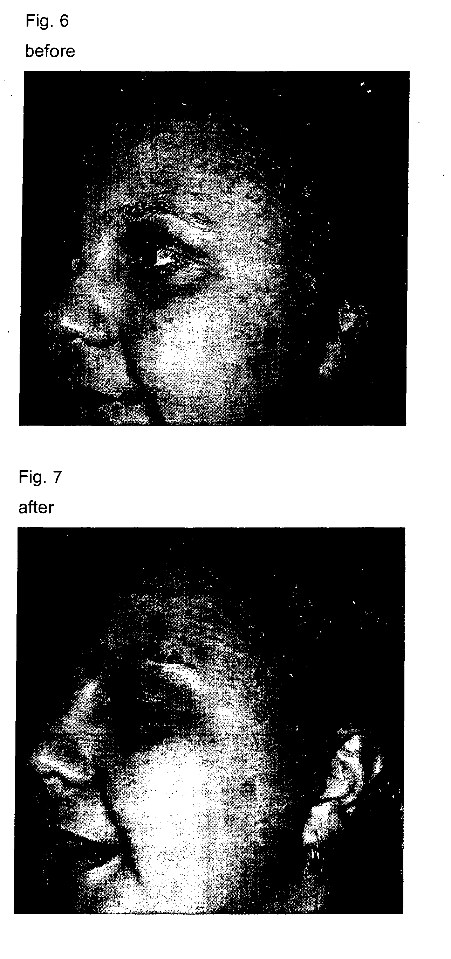

Exemplificative case n° 3: This case concerns the application for cosmetic use of vitamin C (LAA) complexed with melatonin and resveratrol and creams with vitamin A and vitamin E, complexed with melatonin 0.5% resveratrol and polifenols and containing 0.1 % of phytosphingosine. A 26 year old patient, having solar spots on face and "lentigo solaris". Serum object of the present invention is applied with creams containing as active principle, substances such as derivatives of vitamin A, C and E, complexed with melatonin and resveratrol. Whitening action is mainly obtained by serum of vitamin C and partially also by creams containing alpha arbutin up to 10 % stabilized with melatonin, resveratrol and vitis vinifera together with ascorbic acid, LAA cogic acid or azelaic acid. After 8 weeks from the beginning of the treatment, patient is happy, lentigo solaris completely disappeared, cute is soft, well hydrated and with a uniform colour (Fig.5 and 6).

Exemplificative case n° 4: This case concerns the application for cosmetic use of vitamin C complexed with melatonin in combination with creams containing vitamin A, C and derivatives, vitamin E and cogic and/or azelaic acid, complexed with melatonin, resveratrol, Vitis Vinifera and polyphenols together with phytosphingosine. A 78 year old patient, suffering of ageing and actinic keratosis and diffuses teleangectasys on the face. Patient has been treated with creams comprised of the above active substances, complexed with Vitis Vinifera, alpha hydroxyacid, salicylic and malaleuca alternifolia and vitamin C, 15% with melatonin and resveratrol as serum. Patient was subjected to a domiciliary treatment twice a day.

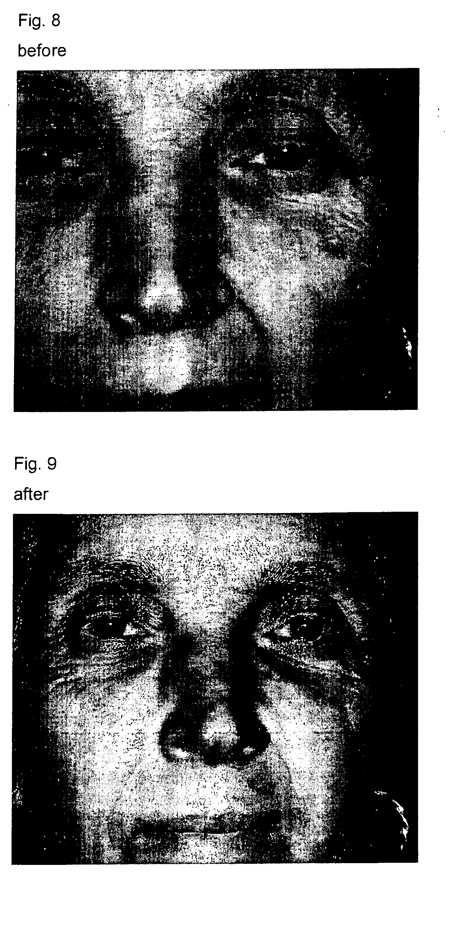

The results were achieved after 3 months of treatment and evident improvement of wrinkles and whitening action of substances used for restoring the colour and the cutaneous tonicity (Fig. 7 and 8).

Action of LAA complexed with Melatonin and resveratrol toghehter with Vitis Vinifera, not only in serum form but also as creams, comprising vitamin C, vitamin A and their derivatives, vitamin E, ascorbic acid and azelaic acid, Arbutin and Fitic acid, all complexed with resveratrol, Vitis Vinifera and melatonin. Particularly, action of Vitis Vinifera and/or melatonin and or resveratrol when complexed, is synergic in efficiency and action for:
- prevention and treatment of spots (preventive action when exposed to sun rays, and in the post-inflammatory pigmentation, PIH);
- treatment and prevention of skin tumours, lentigo senilis, lentigo solaris, etc.;
- treatment of actinic keratosis;
- treatment of surgical wounds since the whole participates in the healing processes;
- restoring of cutaneous functionality and homeostasis;
- restoring and stimulation of normal cellular hydration;
- restoring of regular cutaneous elasticity and of functions relevant to collagen action;
- treatment of bedsores, diabetic cutaneous sores and so on;
- treatment of oral mucous lesions;
- cutaneous and mucous reepithelialization processes;
- treatment of acne;
- treatment of bum sores;
- treatment of surgical wounds also when combined with use of collagen where collagen exerts also a support action to the process of reepithelialization stimulated by vitamin C complexed with Resveratrol and Melatonin;
- treatment of surgical accidents in intestinal surgery when it is wished preventing the beginning of cicatricial bridles, since said substances are modulators of the collagen synthesis;
- treatment of skin diseases in cases when it is possible providing the use of substances containing vitamin C, A, E, etc. complexed with resveratrol and melatonin and Vitis Vinifera;
- treatment of cicatricial and acute consequences of histic infarct, also cardiac infarct, being the substances important mediators for healing;
- treatment of oral mucous lesions, induing aphtas and herpes;
- treatment of cutaneous herpes.

Compositions according to the present invention exert their action both on the nervous system, stimulating a repair of nerves both on the epithelial tissues where surely complex with vitamin C and Melatonin plays a key role particularly for creation of collagen.

It has been demonstrated that both vitamin C and resveratrol and melatonin and Vitis Vinifera extract have an action in preventing skin tumours

Furthermore, it has been noticeably noted that complex of melatonin, for its antiserotoninergic action, combined with resveratrol and vitis vinifera inhibit and reduce collateral effects due to the use only of vitamin A and derivatives, such as new-production of vessels, cutaneous teleangectasys surely inducing unaesthetism. Said protection of the membrane vessel is due to the action of complex vitamin C - melatonin and resveratrol or vitaminC Vitis Vinifera extract and this clinically noted on patients having a cute with a perfect vascularisation, hydration, where presence of telengactasys is irrelevant.

Considering the action of vascular protection of complex LAA - Vitis Vinifera - and/or melatonin and/or polyphenols and/or resveratrol, the latter has its maximum therapeutically result in association with tretinoine complexed with ionene and/or olea when treating rosacea, i.e. a case with a rich vascular component.

It is further to be noted that complex LAA - Vitis Vinifera and/or polyphenols and or resveratrol accelerates healing processes on skin exfoliated following to a peeling.

### EXAMPLE 6: Composition comprising melatonin, Vitis Vinifera and vitamin C and resveratrol and vitamin A and vitamin E according to the invention

The following concentrations are given in weight %:

### (wording is lnci code recognized and used for each product internationally)

| | % | per kg |
|---|---|---|
| AQUA | | 500 |
| AS | | 0,2 |
| GLICEROLO NAT. | | 10 |
| ACEFLUID | | 2 |
| silicol 350 | | 10 |
| AMIGDALOL | | 10 |
| FATTILAN V | | 110 |
| OLIO DI MANDORLE DOLCI | | 30 |
| MIRISTATO ISOPROPILE | | 80 |
| tegosoft op | | 80 |
| OLIO VINACCIOLI | | 50 |
| OLIO DI MACADAMIA | | 20 |
| BHT | | 5 |
| simulgel | | 4 |
| mandorle e. g. | | 30 |
| VITE ROSSA E.G. | 3 | 30 |
| aloe acef | | 1,1 |
| PHENOXYETHANOLO | | 3 |
| VALINA | | 2 |
| ISOLEUCINA | | 3 |
| LEUCINA | | 3 |
| LISINA | | 2 |
| ARGININA CLH | | 2 |
| taurine | | 2 |
| tirosina | | 2 |
| ESCINA | 0,04 | 0,4 |
| PIRIDOSSINA B6 | 0,3 | 3 |
| bont peptide | 0,3 | 3 |
| FITOSFINGOSINE | 0,2 | 0,2 |
| RESVERATROLO | | 0,2 |
| IMIDAZOLIDINIL UREA | 0,15 | 1,5 |
| sodio deidroacetato | 0,15 | 1,5 |
| SODIO ASCORBIL FOSFATO | 1 | 2 |
| vit c | 1 | 2 |
| TAURINA | 0,5 | 1 |
| VIT A | | 4 |
| VIT E | | 5 |
| SODIO JALURONATO | | 0,4 |
| RIBOFLAVINA B2 | | 0,014 |
| PANTOTENATO CALCIO B5 | | 1 |
| MELATONINA | 0,5 | 5 |
| PROF. UVA | | 2 |
| ACIDO LATTICO | | 12 |
| tinovis | | 1,6 |

### EXAMPLE 7: Composition comprising melatonin complexed with alpha arbutine, and resveratrol and Vitis Vinifera extracts and vitamin A and vitamin E according to the invention

The following concentrations are given in weight %:

| ingredienti | % |
|---|---|
| aqua | 34 |
| ac. Azelaico | 10 |
| xantam gum | 0,25 |
| glicerina | 1,25 |
| transcutol gc | 5 |
| edta bisodico | 0,01 |
| amigdalol | 1 |
| olio macadamia | 6 |
| olio vinaccioli | 6 |
| isopropil miristato | 3 |
| cetilico 3 m | 3 |
| fattilan v | 8 |
| bht | 0,01 |
| Phytosfingosine | 0.2 |
| aqua | 10 |
| vit c asc | 2 |
| alfa arbutina | 10 |
| melatonina | 1 |
| actinibio ac | 0,3 |
| phenoxyetanolo | 0,1 |
| sodium deydroacetate | 0,1 |
| ac. Glicolico | 5 |

## Claims

1. Compositions comprising one or more vitamins and/or hormons and/or alpha arbutine and/or their derivatives in association with a Vitis Vinifera extract, preferably an aqueous extract, and/or one or more of its components, such as resveratrol, polyphenols and anthocyanidine, and/or ionene polymers, along with one or more adjuvants and/or excipients pharmaceutically or cosmetically acceptable.

2. Compositions according to claim 1, wherein said vitamins and/or their derivatives are ascorbic acid and/or its derivatives.

3. Compositions according to claim 2, wherein said ascorbic acid is L-ascorbic acid, and/or wherein derivatives of ascorbic acid are esters of ascorbic acid with fatty acids or their salts, and/or ester of ascorbic acid with fatty acids is ascorbyl palmitate.

4. Compositions according to claim 1, wherein vitamins are chosen in the group comprising vitamin A, E, D, K, B1, B2, B3, B5, B6, B8, B9, B12, and/or wherein hormons are chosen in the group comprising melatonin, αMSH, testosteron, estrogens, pregnenolon, calcitonin, somatostatin.

5. Compositions according to claim 1 to 4, wherein alpha arbutine is stabilized with melatonin, polyphenols, Vitis Vinifera, ascorbic acid and phytosphingosine, and/or further comprising Echinacea purpurea extract, angustifolia or pallida, and/or one or more active principles contained in Echinacea purpurea extract, angustifolia or pallida, such as heteroxylanes, rhamnoarabino-galactanes, glycoproteines, cichoric acid, alkylamides, caffeic acid derivatives, flavonoids, caryophyllene and green tea.

6. Compositions according to claim 5, wherein derivatives of caffeic acid are echinacoside or Echinacea and/or wherein flavonoid is rutin.

7. Compositions according to one of the preceding claims, further comprising one or more compounds chosen from the group comprising polymeric ethers, monohydric alcohols, polyhydric alcohols, tetraborates or thiosulfates.

8. Compositions according to claim 7, wherein polymeric ethers are ethylene polymers or propylene oxides and/or wherein polyhydric alcohol is ethoxydiglycol.

9. Use of compositions according to claims 1 - 8 for preparation of a medicament for treatment of keratosis actinic and/or for treatment of wounds and/or for treatment of sores and/or for treatment of diabetic cutaneous sores and/or of acute and chronic lesions of skin and/or for treatment of lesions of oral mucous and/or for prevention and treatment of skin tumours and/or for treatment of psoriasis and/or for treatment of hair grow as skin tone balancing cream and/or as modulator of the TNF and inflammatory processes and/or for treatment of the post inflammatory hyperpigmentation melasma and/or for treatment of laser complications, and/or for treatment of cellulites and striae (strech masks).

10. Use of resveratrol and/or melatonin and/or polyphenols and/or ionene polymers and/or Vitis Vinifera for stabilising compositions comprising vitamins, and/or wherein compositions are aqueous solutions or emulsions and/or wherein vitamins are chosen from the group comprising vitamin A, B1, B2, B3, B5, B6, B8, B9, B12, C, D, E, K and/or aminoacids as well as oligopeptides.
